# EUROPEAN PATENT SPECIFICATION

(11) **EP 4 192 376 B1**
(45) Date of publication and mention of the grant of the patent: **25.11.2026**
(21) Application number: 21856600.8
(22) Date of filing: 10.08.2021
(51) Int. Cl.: A61B 17/88, A61F 2/46, A61F 2/08, A61F 2/28, A61L 27/36, A61L 29/14

(54) **TISSUE GRAFT DELIVERY DEVICES**
GEWEBETRANSPLANTATEINFÜHRUNGSVORRICHTUNGEN
DISPOSITIFS D'ADMINISTRATION DE GREFFON TISSULAIRE

(30) Priority: 10.08.2020 US 202063063812 P
(43) Date of publication of application: 14.06.2023
(73) Proprietor: Lifenet Health, Virginia Beach, VA 23453 (US)
(72) Inventor: ENTSMINGER, Adam, Virginia Beach, VA 23453 (US); BERGER, Cheryl, Virginia Beach, VA 23453 (US); MASTERS, Michael, Virginia Beach, VA 23453 (US); SHORES, Kevin, Virginia Beach, VA 23453 (US); LOHMANN, Erich, Virginia Beach, VA 23453 (US); CHEN, Jingsong, Virginia Beach, VA 23453 (US)
(74) Representative: Scheele, Friedrich
(86) International application number: PCT/US2021/045441
(87) International publication number: WO 2022/035899

(56) References cited:
- WO-A1-2018/232100
- US-A1- 2007 055 283
- US-A1- 2007 185 496
- US-A1- 2011 082 424
- US-A1- 2012 116 515
- US-B2- 10 231 846
- US-B2- 9 265 524

## Description

### CROSS-REFERENCE TO RELATED APPLICATIONS

This application claims the benefit of U.S. Provisional Application No. 63/063,812, filed August 10, 2020.

### FIELD OF THE INVENTION

The invention relates generally to devices for delivering tissue grafts to treatment sites, especially in a minimum invasive surgery (MIS), and uses thereof.

### BACKGROUND OF THE INVENTION

In recent years minimally invasive surgery (MIS) has been well developed, due to the innovation and advancement of surgical instruments and techniques. MIS refers to any procedure that is less invasive than open surgery. Assisted by video instruments and specialized retractors, surgeons can perform complex MIS surgical procedures such as lumbar discectomy, laminectomy, and intervertebral fusion. This often causes less harm to nearby muscles and other tissues. It can also lead to lower levels of blood loss, less pain, and faster recovery after surgery. To achieve a spinal fusion, surgeons need to implant bone grafts into the narrow intervertebral space through a funnel or a tubular device. The surgeons often need to load the bone graft into a funnel or tubular device on the back table in the operation room, which can be time consuming. Furthermore, the bone graft frequently jams in the device during the injection process which can be very challenging for the surgeons to overcome during the surgical procedure. Thus, it is very desirable to develop a product of a cannula pre-loaded with bone graft, which will minimize or eliminate jamming during the injection process.

US2007/055283 A1 illustrates for example an apparatus for introducing material into bone through a subcutaneous cannula, the apparatus including a subcutaneous cannula, a delivery device to convey the material at a delivery pressure of no greater than about 25 bar (360 psi), a nozzle instrument capable of advancement into the subcutaneous cannula and comprising a proximal fitting to couple the nozzle instrument to the delivery device and a nozzle terminus through which the material conveyed by the delivery device enters bone at the delivery pressure, and a tamping instrument capable of advancement into the subcutaneous cannula and having a tamping terminus which, during the advancement, urges material residing in the subcutaneous cannula into bone
WO 2018/232100 A1 relates to a medical implant comprises a slurry of bone particles that are injected into a vertebral body under pressure. The liquid component of the slurry may be aspirated while the slurry is being injected so that the bone particles of the slurry pack into the central area of the vertebral body to provide structural support. The injected slurry may be agitated during the procedure to maximize the structural strength of the implant after the procedure.

US 2012/116515 A1 concerns implants comprising a plurality of separate cortical bone units, which have been at least partially demineralized and are osteoinductive, are described herein. The implants can be used in methods for treating bone. Also, disclosed are methods for treating spinal conditions using these implants. The spinal conditions include but are not limited to repairing damage to or defects in the spine, such as fractures in a vertebral body or degeneration of spinal discs.

### SUMMARY OF THE INVENTION

The invention is defined in the independent claims. Further advantageous embodiments are defined in the dependent claims.

### BRIEF DESCRIPTION OF THE DRAWINGS

Fig. 1 illustrates a partial and broken cross-section view of a loading device and the variables associated with calculating the spring force restraining a cannula. According to the figure, k₁, k₂ are the spring constants (N/mm : lb/in), x₀ is the uncompressed spring length (mm : in), x₁ is the compression distance caused by cannula (mm : in) and x₂ is the minimum compression distance to remove cannula (mm : in).
Fig. 2 shows a test fixture to restrain and actuate a delivery device while measuring extrusion force with a load cell.

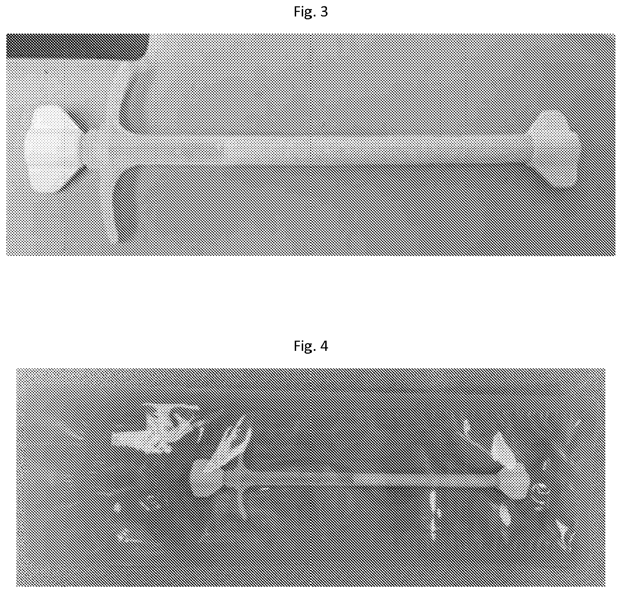

Fig. 3 shows a cannula with a tissue graft according to one embodiment of the present invention.
Fig. 4 shows a cannula with a tissue graft in representative commercial packaging according to another embodiment of the present invention.
Fig. 5 shows high frequency Instron load cell measurements of handle resistance during cannula extrusion of a sample tissue graft using a delivery device and Instron test fixture. Each peak in the force measurement corresponds to a pull of the device handle, advancing the push rod down the cannula.
Fig. 6 shows estimated maximum force of the push rod on the tissue at stroke (N) over stroke number 1 through 16 during extrusion from a delivery device for several test articles. The mechanical advantage for this example was estimated as 3.8 times the measured extrusion force applied to the device handle and used to scale the data recorded by the Instron test fixture in Fig. 2. The plot shows variations from cannula to cannula, which includes variations from donor to donor and loading technician to loading technician.

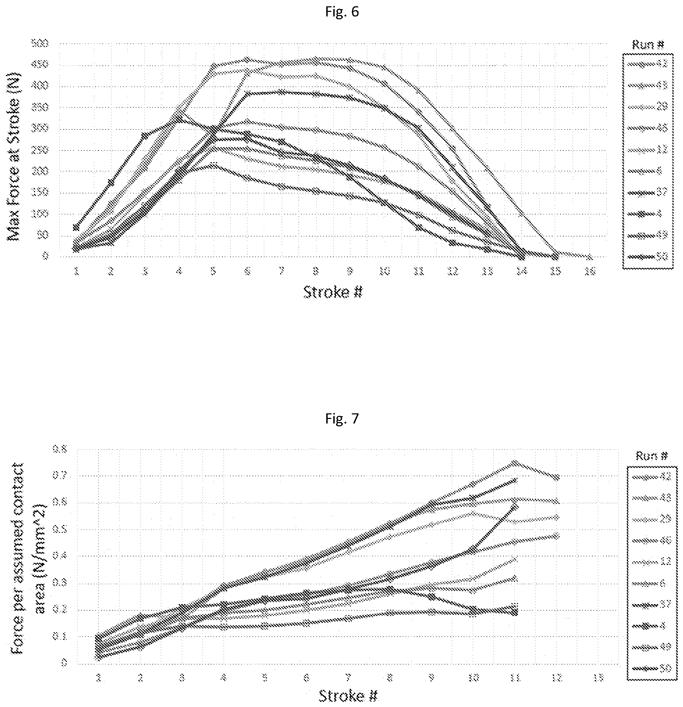

Fig. 7 shows force per assumed contact area (N/mm²) over stroke number 1 through 13. This data takes the data points in Fig. 6 and makes assumptions about the amount of the tissue graft remaining in the cannula during that stroke (i.e., the last stroke recorded, which has very little resistance force, is assumed to be empty and so the stroke prior to that has approximately 0.25 cc (i.e., cm³) tissue based on the spacing of teeth on the push rod in the delivery device. Working backwards from the last stroke, the length of the graft in the cannula may be determined and therefore the surface area in contact with the cannula at each stroke). The peak force from Fig 6 divided by the assumed surface area of contact between the graft and cannula may be calculated to show that the friction force is likely increasing, possibly due to tissue graft compression and increased force on the cannula walls, even as the tissue graft is being extruded from the cannula.
Fig. 8 shows a rendered depiction of a cannula, which has been pre-filled with a tissue graft, according to one embodiment of the present invention.

### DETAILED DESCRIPTION OF THE INVENTION

The present invention provides a cannula or delivery system for delivering a tissue graft to a treatment site as well as uses thereof, especially in a minimum invasive surgery (MIS). The invention is based on the inventors' surprising discovery of a novel semi-automatic bone particulate loading device and a novel method capable of controlling a loading density and extrusion force of a loaded cannula. The inventors have discovered that loading a tissue plug into a cannula from the distal end minimizes friction effects and compression of the resulting tissue graft in the cannula because the travel distance of the tissue plug is minimized during loading. The inventors have also unexpectedly discovered that a cannula loaded with bone cell particulates, such as bone chips, optionally with or without demineralized bone matrix (DBM), maintains viable bone cells and cell activities while providing improved surgical procedures and outcomes. The newly discovered MIS allograft delivery device provides further enhanced new bone formation, reduced extrusion resistance, higher allograft yield per donor, and lower cost of goods sold (COGS).

The term "cannula" used herein refers to a thin tube used in a surgery for delivering a substance to a treatment site in a subject. The cannula has an exterior surface and an interior surface. The exterior surface defines the external diameter of the cannula. The interior surface forms a bore extending along the axis of the cannula and defines an inner diameter. The bore may be a cylinder.

The term "tissue graft" used herein refers to a tissue to be transplanted surgically into a subject at a treatment site. The tissue graft may be prepared from any tissue.

The term "treatment site" used herein refers to a site of defect in the body of a subject in need for treatment. The treatment site may be on the surface of the body or in a body cavity.

The term "constant diameter" used herein refers to the diameter of an elongated object along its axis having a variation no more than about 1%, 2%, 3%, 4%, 5%, 6%, 7%, 8%, 9% or 10%. For example, a cannula may have a constant inner diameter.

The term "delivery device" used herein refers to an apparatus for extruding a tissue graft from a cannula to a treatment site. The delivery device may comprise two or more components. The delivery device may comprise a connector with threaded ends for easy assembly with and disassembly from the cannula. A delivery system may be formed by a delivery device and a cannula.

The term "push rod" used herein refers to a component of a delivery device, which may be actuated directly or indirectly through one or more mechanisms to extrude a tissue graft from the cannula. The push rod may be stiff or flexible, and may take on any shape, for example, a cylinder. The push rod may have features that allow actuation of less than the full length of the push rod or cannula.

The term "push handler" used herein refers to a component of a delivery device, which may restrain a cannula and may contain one or more mechanisms to actuate the push rod to extrude a tissue graft from the cannula.

The term "stroke" used herein refers to one cycle of actuation of a push rod, where the push rod may advance from the proximal end of the cannula towards the distal end of the cannula at any increment of distance.

The term "mechanical advantage" used herein refers to a force amplification achieved by a delivery device using its components via one or more mechanisms. The components may include a push rod, push handle, lever, gear, chain drive, belt drive, pneumatic, hydraulic or a combination thereof. For example, the mechanical advantage developed in a delivery device may be generated through a lever mechanism, which is pinned in the middle for rotation with a longer push handle on one side and a shorter push rod advancement mechanism on the other side.

The term "maximum extrusion force" used herein refers to the maximum force applied to actuate or engage a delivery device to extrude a tissue graft at any time during extrusion of the tissue graft from the cannula. The maximum extrusion force applied to the delivery device may introduce a mechanical advantage, such that the force experienced by the tissue graft from the push rod is higher than the applied maximum extrusion force. The extrusion of a tissue graft may be completed over one or more strokes of the push rod. The maximum extrusion force should not exceed an upper limit whereby the tissue graft remains in its desirable condition and shape and remains in a desired treatment site after being expelled from the cannula to the treatment site.

The term "tissue plug" or "tissue column" used herein interchangeably and refer to an aliquot of a tissue that is prepared or preformed in a predetermined size and shape. One, two, or more tissue plugs or tissue columns may be used for forming a tissue graft. The tissue plug or tissue column may be prepared by conventional techniques.

The term "loading device" used herein refers to an apparatus used to load a tissue plug into a cannula.

The term "void" refers to a portion of a bore in a cannula intended to be filled with a tissue graft. An empty cannula may be placed in a loading device, and a physical tissue backstop may be placed into the bore from its proximal end to prevent a tissue plug from going past the tissue backstop. The physical tissue backstop may be a dowel with a slightly smaller diameter than the inner diameter of the cannula. The tissue backstop allows air and liquid to pass, but not the tissue plug, and creates a defined void volume to be filled by a tissue plug while preventing buildup of pressure as the tissue plug is pushed into the cannula.

The term "tissue density" used herein refers to the mass of a tissue graft in a cannula divided by the void volume, where the tissue graft may be in any form.

The term "particulate density" used herein refers to the mass of a tissue graft in the cannula divided by the void volume, where the tissue graft is in the form of particulates.

The term "final compression force" used herein refers to the force applied to one end of a tissue plug in a cannula towards the distal end of the cannula during loading, either directly or indirectly by a loading device, and the tissue plug has in turn translated the force to the other end of the tissue plug against the tissue backstop or another tissue plug previously loaded into of the void. The final compression force may be used to establish a consistent tissue graft volume and density while maintaining the extrusion force not exceeding the maximum extrusion force.

The term "final loading pressure" used herein refers to the final compression force transmitted to a tissue plug inside a cannula divided by the cross-sectional area defined by the inner diameter of the cannula.

The term "surface roughness" used herein refers to a quantified deviation of a surface from its ideal form and can be assessed through measurements in combination with the equation below: ${x}_{RMS} = \sqrt{\frac{1}{n} \left({x}_{1}^{2}+{x}_{2}^{2}+⋯+{x}_{n}^{2}\right)} .$ (I) where x is a measurement of the surface height and n is the number of measurements taken. The surface roughness may be measured by using a contact profilometer.

A cannula for delivering a tissue graft to a treatment site is provided. The cannula comprises a distal end, a proximal end, a bore, and a tissue graft. The bore is formed by an interior surface of the cannula, extends from the distal end to the proximal end, and has an inner diameter, which may be constant. The tissue graft is inside the bore and in contact with the interior surface. The tissue graft is released from the cannula at the distal end to the treatment site. The tissue graft has a predetermined final loading pressure, defined by a final compression force on the tissue inside the cannula divided by the cannula bore cross-sectional area. The predetermined final loading pressure may be about 0.07-68.95, 0.69-68.95, 1.38-68.95, 3.45-68.95, 6.89-68.95, 0.07-34.47, 0.69-34.47, 1.38-34.47, 3.45-34.47, 6.89-34.47, 0.07-27.58, 0.69-27.58, 1.38-27.58, 3.45-27.58, 6.89-27.58, 0.07-20.68, 0.69-20.68, 1.38-20.68, 3.45-20.68, 6.89-20.68, 0.07-13.79, 0.69-13.79, 1.38-13.79, 2.07-13.79, 2.76-13.79, 3.45-13.79, 4.14-13.79, 4.83-13.79, 5.52-13.79, 6.21-13.79, 6.89-13.79, 7.58-13.79, 8.27-13.79, 8.96-13.79, 9.65-13.79, 10.34-13.79, 1.38-10.34, 2.07-10.34, 2.76-10.34, 3.45-10.34, 4.14-10.34, 4.83-10.34, 5.52-10.34, 6.21-10.34, 6.89-10.34, 7.58-10.34, 8.27-10.34, 8.96-10.34, 9.65-10.34, 1.38-9.65, 2.07-9.65, 2.76-9.65, 3.45-9.65, 4.14-9.65, 4.83-9.65, 5.52-9.65, 6.21-9.65, 6.89-9.65, 7.58-9.65, 8.27-9.65, 8.96-9.65, 1.38-8.96, 2.07-8.96, 2.76-8.96, 3.45-8.96, 4.14-8.96, 4.83-8.96, 5.52-8.96, 6.21-8.96, 6.89-8.96, 7.58-8.96, 8.27-8.96, 1.38-8.27, 2.07-8.27, 2.76-8.27, 3.45-8.27, 4.14-8.27, 4.83-8.27, 5.52-8.27, 6.21-8.27, 6.89-8.27, 7.58-8.27, 1.38-7.58, 2.07-7.58, 2.76-7.58, 3.45-7.58, 4.14-7.58, 4.83-7.58, 5.52-7.58, 6.21-7.58, 6.89-7.58, 1.38-6.89, 2.07-6.89, 2.76-6.89, 3.45-6.89, 4.14-6.89, 4.83-6.89, 5.52-6.89, 6.21-6.89, 1.38-6.21, 2.07-6.21, 2.76-6.21, 3.45-6.21, 4.14-6.21, 4.83-6.21, 5.52-6.21, 1.38-5.52, 2.07-5.52, 2.76-5.52, 3.45-5.52, 4.14-5.52, 4.83-5.52, 1.38-4.83, 2.07-4.83, 2.76-4.83, 3.45-4.83, 4.14-4.83, 1.38-4.14, 2.07-4.14, 2.76-4.14, 3.45-4.14, 1.38-3.45, 2.07-3.45, 2.76-3.45, 1.38-2.76, 2.07-2.76 bar (1-1,000, 10-1,000, 20-1,000, 50-1,000, 100-1,000, 1-500, 10-500, 20-500, 50-500, 100-500, 1-400, 10-400, 20-400, 50-400, 100-400, 1-300, 10-300, 20-300, 50-300, 100-300, 1-200, 10-200, 20-200, 30-200, 40-200, 50-200, 60-200, 70-200, 80-200, 90-200, 100-200, 110-200, 120-200, 130-200, 140-200, 150-200, 20-150, 30-150, 40-150, 50-150, 60-150, 70-150, 80-150, 90-150, 100-150, 110-150, 120-150, 130-150, 140-150, 20-140, 30-140, 40-140, 50-140, 60-140, 70-140, 80-140, 90-140, 100-140, 110-140, 120-140, 130-140, 20-130, 30-130, 40-130, 50-130, 60-130, 70-130, 80-130, 90-130, 100-130, 110-130, 120-130, 20-120, 30-120, 40-120, 50-120, 60-120, 70-120, 80-120, 90-120, 100-120, 110-120, 20-110, 30-110, 40-110, 50-110, 60-110, 70-110, 80-110, 90-110, 100-110, 20-100, 30-100, 40-100, 50-100, 60-100, 70-100, 80-100, 90-100, 20-90, 30-90, 40-90, 50-90, 60-90, 70-90, 80-90, 20-80, 30-80, 40-80, 50-80, 60-80, 70-80, 20-70, 30-70, 40-70, 50-70, 60-70, 20-60, 30-60, 40-60, 50-60, 20-50, 30-50, 40-50, 20-40 or 30-40 psi). For example, the predetermined final loading pressure may be 4.34 - 7,65 bar (63-111 psi).

The cannula may be used with a delivery device for delivering a tissue graft in a surgery, for example, a minimum invasive surgery, in which the treatment site may have a small incision.

A cannula may be selected for its outer diameter (OD) (e.g., 8mm) to be about the maximum desired to fit inside the annulotomy of the treatment site, for example, between the vertebrae. The OD of the cannula may be no more than 30, 25, 20, 15, 14, 13, 12, 11, 10, 9, 8, 7, 6 or 5 mm. The selection of the inner diameter (ID) (e.g., 6mm) may be driven by structural factors and manufacturing limitations for wall thickness. A larger ID is better for delivering a graft material during expression from the cannula, or while the graft material is coming out of the cannula (e.g., being extruded), but the limit is fitting the cannula (OD) into the surgical site (such as the intervertebral disk space). The ID of the cannula may be 1-25, 1-20, 1-15, 1-10, 1-9, 1-8, 1-7, 1-6, 1-5, 2-25, 2-20, 2-15, 2-10, 2-9, 2-8, 2-7, 2-6, 2-5, 3-25, 3-20, 3-15, 3-10, 3-9, 3-8, 3-7, 3-6, 3-5, 4-25, 4-20, 4-15, 4-10, 4-9, 4-8, 4-7, 4-6, 4-5, 5-25, 5-20, 5-15, 5-10, 5-9, 5-8, 5-7, or 5-6 mm.

The cannula may have threaded ends allowing a cap to be easily assembled and disassembled at either end to help keep the tissue graft in the cannula. The delivery device may contain female threading to accept the threaded end of the cannula, an interface to support and direct the delivery device and cannula, such as a handle, and a mechanism for actuation to extrude a tissue graft from a cannula, such as a trigger that acts a lever to advance a push rod.

The tissue graft may be an allograft, autograft, xenograft or a combination thereof. The tissue graft may comprise bone, cartilage, soft tissue, placental tissue, birth tissue, umbilical cord, vascular tissue, tendon, ligament, fascia, dermis, periosteum, adipose, or a combination thereof.

The tissue graft may be in a form of particulates, putty, powder, gel, slurry, sponge, or a combination thereof. The tissue graft may be frozen, plasticized, cryopreserved or freeze dried.

The cannula may have a particulate density of 0.1-10, 0.1-5, 0.1-1, 0.1-0.5, 0.5-10, 0.5-5, 0.5-1, 1-10, 1-5, 1-4, 1-3, 1-2, 1-1.5, 1-1.4, 1-1.3, 1-1.2, 1-1.1, 1.1-5, 1.1-4, 1.1-3, 1.1-2, 1.1-1.5, 1.1-1.4, 1.1-1.3, 1.1-1.2, or 5-10 g/cm³, less than 10, 5, 1, 0.5 or 0.1 g/cm³, or no more than 0.5, 1, 5 or 10 g/cm³. In one embodiment, the particulate density may be less than 5.0 g/cm³. In another embodiment, the particulate density may be 1.1-1.3 g/cm³.

The tissue graft may have a maximum extrusion force of 100-300, 100-250, 100-200, 100-150, 150-300, 150-250, 150-200, 160-200, 160-190, 160-180, 160-170, 170-200, 170-190, 170-180, 180-200, 180-190, 190-200, 200-300, 200-250 or 250-300 N, less than 150, 160, 170, 180, 190, 200, 250 or 300 N, or more than 100, 150, 160, 170, 180, 190, 200, 250 or 300 N. In one embodiment, the maximum extrusion force may be 180-190 N (e.g., 187 N) or 150-160 N (e.g., 152 N).

The cannula may be loaded with a tissue density at 0.01-20, 0.01-15, 0.01-10, 0.01-5, 0.01-1, 0.01-0.5, 0.01-0.1, 0.01-0.05, 0.05-20, 0.05-15, 0.05-10, 0.05-5, 0.05-1, 0.05-0.5, 0.05-0.1, 0.1-20, 0.1-15, 0.1-10, 0.1-5, 0.1-1, 0.1-0.5, 0.2-20, 0.2-15, 0.2-10, 0.2-5, 0.2-1, 0.2-0.5, 0.5-20, 0.5-15, 0.5-10, 0.5-5, 0.5-1, 1-20, 1-15, 1-10, 1-5, 1-4, 1-3, 1-2, 1-1.5, 1-1.4, 1-1.3, 1-1.2, 1-1.1, 5-20, 5-15, 5-10, 10-20, 10-15, or 15-20 g/cm³, at least 0.01, 0.05, 0.1, 0.2, 0.5, 1, 5, 10 or 15 g/cm³, or no more than 0.05, 0.1, 0.2, 0.5, 1, 5, 10, 15 or 20 g/cm³.

The cannula may comprise viable cells. There may be more than 1,000, 5,000, 10,000, 11,000, 12,000, 13,000, 14,000, 15,000, 16,000, 17,000, 18,000, 19,000, 20,000, 25,000, 30,000, 50,000, 100,000, 200,000, 300,000, 400,000, 500,000 or 1,000,000 viable cells/cm³ of the tissue graft in the cannula. At least 40, 50, 60, 70, 80, 90, 95 or 99 % of the viable cells remain viable after the cannula is stored for a predetermined time, for example, at least 1, 2, 3, 4, 5, 6, or 7 days, 1, 2, 3, 4, 5, 6, 7, or 8 weeks, or 1, 2, 3, 4, 5, 6, 9, 12, 18 or 24 months. The viable cells may be naturally occurring cells in the tissue from which the tissue graft is prepared, with or without culture. The viable cells may be seeded to the tissue graft, with or without further culture expansion. The viable cells may be differentiated cells, progenitor cells or stem cells from the tissue. The viable cells may be progenitor cells or differentiated cells derived from stem cells from the tissue.

The tissue graft may be a bone graft. The bone graft may comprise viable bone cells or bone forming cells. There may be more than 1,000, 5,000, 10,000, 11,000, 12,000, 13,000, 14,000, 15,000, 16,000, 17,000, 18,000, 19,000, 20,000, 25,000, 30,000, 50,000, 100,000, 200,000, 300,000, 400,000, 500,000 or 1,000,000 viable bone cells or bone forming cells per cm³ of the bone graft. In one embodiment, the bone graft may comprise more than 16,000 viable bone cells or bone forming cells. At least 40, 50, 60, 70, 80, 90, 95 or 99 % of the viable bone cells or bone forming cells remain viable after the cannula is stored for a predetermined time, for example, at least 1, 2, 3, 4, 5, 6, or 7 days, 1, 2, 3, 4, 5, 6, 7, or 8 weeks, or 1, 2, 3, 4, 5, 6, 9, 12, 18 or 24 months. The viable cells may be naturally occurring cells in the bone from which the bone graft is prepared, with or without being cultured. The viable cells may be seeded to the bone graft, with or without further culture expansion. The viable cells may be differentiated cells, progenitor cells or stem cells from the tissue. The viable cells may be progenitor cells or differentiated cells derived from stem cells from the bone.

The cannula may further comprise the cryopreservation solution in the amount of 0.5-2.5, 0.5-2, 0.5-1.5, 0.5-1, 1-2.5, 1-2, 1-1.5, 1.5-2.5, 1.5-2 or 2-2.5 ml (cc), or about 10-90, 10-80, 10-70, 10-60, 10-50, 10-40, 10-30, 10-20, 20-90, 20-80, 20-70, 20-60, 20-50, 20-40, 20-30, 30-90, 30-80, 30-70, 30-60, 30-50, 40-90, 40-80, 40-70, 40-60, 40-50, 50-90, 50-80, 50-70, 50-60, 60-90, 60-80, 60-70, 70-90, 70-80 or 80-90 % by volume, based on the total volume of the void in the cannula. The tissue may be saturated with the cryopreservation solution prior to loading. The volume ratio of the cryopreservation solution to the tissue graft may be 0.1-10, 0.1-5, 0.1-1, 0.1-0.5, 0.5-10, 0.5-5, 0.5-1, 1-10, 1-5, or 5-10, less than 0.1, 0.5, 1, 5 or 10, or more than 0.1, 0.5, 1, 5 or 10.

The bone graft may comprise bone particulates. The bone particulates may have viable bone cells. The bone particulates may be nondemineralized bone chips, which may be cortical bone chips, corticocancellous bone chips, cancellous bone chips, or a combination thereof. The volume ratio of the cryopreservation solution to the bone graft, for example, bone particulates, may be 0.1-10, 0.1-5, 0.1-1, 0.1-0.5, 0.5-10, 0.5-5, 0.5-1, 1-10, 1-5, or 5-10, less than 0.1, 0.5, 1, 5 or 10, or more than 0.1, 0.5, 1, 5 or 10.

The tissue graft may be an allograft. The allograft may be a bone allograft formulated from morselized nondemineralized corticocancellous bone, cortical bone, cancellous bone, or mixtures thereof containing native lineage committed bone cells (live bone) and demineralized cortical bone matrix (DBM) to promote new bone formation. Other allografts may contain native growth factors that promote healing and provide clinical benefit. Other tissue types that may be suitable for the delivery system include most allografts from viscous liquids to granular compressible solids. The bone graft may exclude DBM.

The tissue graft may comprise a bone graft comprising nondemineralized corticocancellous bone and demineralized ground cortical bone. The corticocancellous bone and the demineralized ground cortical bone may have a volume ratio from 10:1 to 1:10, from 5:1 to 1:5, from 5:1 to 1:4, from 5:1 to 1:3, from 5:1 to 1:2, from 5:1 to 1:1, from 5:1 to 2:1, from 5:1 to 3:1, from 5:1 to 4:1, from 4:1 to 1:5, from 4:1 to 1:4, from 4:1 to 1:3, from 4:1 to 1:2, from 4:1 to 1:1, from 4:1 to 2:1, from 4:1 to 3:1, from 3:1 to 1:5; from 3:1 to 1:4, from 3:1 to 1:3, from 3:1 to 1:2, from 3:1 to 1:1, from 3:1 to 2:1, from 2:1 to 1:5, from 2:1 to 1:4, from 2:1 to 1:3, from 2:1 to 1:2, from 2:1 to 1:1, from 1:1 to 1:5, from 1:1 to 1:4, from 1:1 to 1:3 or from 1:1 to 1:2. In some embodiments the nondemineralized corticocancellous bone includes corticocancellous bone, cortical bone, cancellous bone, or mixtures thereof.

The corticocancellous bone, cortical bone, cancellous bone, or mixtures thereof may comprise viable bone cells or bone forming cells. The corticocancellous bone may have a particle size of 0.1-40, 0.1-30, 0.1-20, 0.1-10, 0.1-5, 0.1-4, 0.1-3, 0.1-2, 0.1-1, 0.5-40, 0.5-30, 0.5-20, 0.5-10, 0.5-5, 0.5-4, 0.5-3, 0.5-2, 0.5-1, 1-40, 1-30, 1-20, 1-10, 1-5, 1-4, 1-3, 1-2, 2-40, 2-30, 2-20, 2-10, 2-5, 2-4, 2-3, 3-40, 3-30, 3-20, 3-10, 3-5, 3-4, 4-40, 4-30, 4-20, 4-10, 4-5, 5-40, 5-30, 5-20, 5-10, 10-40, 10-30, 10-20 or 20-30 mm, at least 0.1, 0.5, 1, 2, 3, 4, 5, 10, 20 or 30 mm, or no more than 0.5, 1, 2, 3, 4, 5, 10, 20, 30 or 40 mm.

The corticocancellous bone, cortical bone, cancellous bone, or mixtures thereof may be demineralized or sintered.

The demineralized ground cortical bone may have residual calcium at 0.1-8, 0.1-5, 0.1-4, 0.1-3, 0.1-2, 0.1-1, 0.5-8, 0.5-5, 0.5-4, 0.5-3, 0.5-2, 0.5-1, 1-8, 1-5, 1-4, 1-3, 1-2, 2-8, 2-5, 2-4, 2-3, 3-8, 3-5, 3-4, 4-8, 4-5, or 5-8 %, more than 0.1, 0.5, 1, 2, 3, 4, 5, or 8 %, or less than 0.5, 1, 2, 3, 4, 4.1, 4.2, 4.3, 4.4, 4.5, 4.6, 4.7, 4.8, 4.9, 5, or 8 %. The demineralized cortical bone may have a particulate size of 1-10,000, 1-5,000, 1-1,000, 1-500, 1-100, 10-10,000, 10-5,000, 10-1,000, 10-500, 10-100, 100-10,000, 100-5,000, 100-1,000, 100-500, 200-10,000, 200-5,000, 200-1,000, 200-500, 250-10,000, 250-5,000, 250-1,000, 250-500, 500-10,000, 500-5,000 or 500-1,000 µm.

The demineralized ground cortical bone may be free of, for example, or may comprise no more than 1, 2, 3, 4, 5, 6, 7, 8, 9 or 10 % by weight, based on the total weight of the demineralized ground cortical bone, a soft tissue, or foreign particles. Examples of the soft tissues include tendon, ligament, vascular tissues, fascia, skin, dermis, adipose, placenta and nerve tissue. Examples of the foreign particles include synthetic materials, metal, tricalcium phosphate, calcium phosphate, hydroxyapatite, calcium sulfate and synthetic polymers. The cannula may further comprise a cryoprotectant. Examples of the cryoprotectants include dimethyl sulfoxide (DMSO), ethylene glycol, glycerol, 2-methyl-2,4-pentanediol (mpd), propylene glycol, sucrose, and trehalose. For example, the demineralized ground cortical bone may be in an aqueous culture media containing DMSO at, for example, 2%, 5%, 10%, 15%, 20% or 30%, and serum albumin at, for example, 5%, 10%, 15%, 20%, 25%, 30%, based on total volume of the culture media. The tissue graft in the cannula may be cryopreserved. The bone graft may comprise demineralized bone matrix (DBM) no more than 5, 4, 3, 2, 1, 0.5 or 0.1 % residual calcium by weight based on the total weight of the bone graft. The bone graft may comprise nondemineralized corticocancellous bone and demineralized ground cortical bone at a volume ratio from 2:1 to 3:1.

In one embodiment, the bone graft may comprise demineralized ground cortical bone and nondemineralized bone chips, wherein the bone chips comprise cortical, corticocancellous, cancellous, or combinations thereof, and the bone chips include viable bone-forming cells.

The cannula may be a syringe cannula. The cannula may be an IV cannula or nasal cannula but without trocar or needle. The cannula may be straight or curved.

The treatment site may be at any location in the body of the subject. For example, the treatment site may be in a spine, dental, foot, jaw, ankle, limb bone or joint. The treatment site may also be a defect site in a soft tissue, cartilage defect, synovial membrane, or spine disc. Where the cannula is used for intervertebral fusion, the treatment site may be around a cage or into a cage, which may be expandable. The treatment site may be in the disc space around or within an interbody cage.

The cannula of the present invention is pre-filled or loaded with a tissue graft. The cannula may be provided with a delivery device. For convenience, a package of two, three or more cannulas and one delivery device is provided in one exemplary embodiment. The product components may be individually packaged and stored, and each cannula and delivery device is intended for a single patient use in accordance with certain aspects of the invention.

The cannula may be useful for spine or general orthopedic applications. The cannula may have printed volume graduations or indicators on the cannula. One or more radiopaque markers may be used for detection of the cannula tip in the disc space using radioscopy. Other functional cannula design modifications may be introduced. For example, tip adapters or threads may be added to facilitate interbody cage filling with allograft.

For each cannula of the present invention, systems for delivering a tissue graft to a treatment site are provided. According to certain aspects of the invention, a system may comprise a cannula and a delivery device. In one embodiment, the delivery device includes a push rod and a push handler. The push rod approaches and contacts the tissue graft in the cannula from the proximal end to extrude tissue at the distal end. The push handler forces the push rod to push the tissue graft out of the cannula at the distal end to the treatment site. The push rod may also be called plunger or racheting plunger.

The delivery device provides a mechanical advantage. The mechanical advantage may be the force amplification generated by the push handler and the push rod. The mechanical advantage may be in the range of 1-10, 1-9.5, 1-9, 1-8.5, 1-8, 1-7.5, 1-7, 1-6.5, 1-6, 1-5.5, 1-5, 1-4.5, 1-4, 1-3.5, 1-3, 1-2.5, 1-2, 2-10, 2-9, 2-8, 2-7, 2-6, 2-5, 2-4, 2-3, 2.5-10, 2.5-9.5, 2.5-9, 2.5-8.5, 2.5-8, 2.5-7.5, 2.5-7, 2.5-6.5, 2.5-6, 2.5-5.5, 2.5-5, 2.5-4.5, 2.5-4, 2.5-3.5, 2.5-3, 3-10, 3-9, 3-8, 3-7, 3-6, 3-5, 3-4, 4-10, 4-9, 4-8, 4-7, 4-6, 4-5, 5-10, 5-9, 5-8, 5-7, 5-6, 6-10, 6-9, 6-8, 6-7, 7-10, 7-9, 7-8, 8-10, 8-9 or 9-10. The mechanical advantage may be greater than 1, 1.5, 2, 2.5, 3, 3.5, 4, 4.5, 5, 5.5, 6, 6.5, 7, 7.5, 8, 8.5, 9, 9.5 or 10.

A method for preparing a cannula for delivering a tissue graft to a treatment site is provided. In one aspect, a method comprises connecting the cannula of the present invention with a delivery device having a push rod. The method may further comprise contacting the push rod with the tissue graft so that the push rod is on the opposite side of the tissue graft from the distal end. Thereafter, but not part of the aforementioned method, the push rod may be forced towards the distal end. As a result, the push rod may push the tissue graft out of the cannula at the distal end to the treatment site.

According to the method of the present invention, the push rod may be configured to push the tissue graft at a pressure of 6.89-344.73, 6.89-310.26, 6.89-275.79, 6.89-241.31, 6.89-206.84, 6.89-172.37, 6.89-137.89, 6.89-103.42, 6.89-68.95, 6.89-34.47, 34.47-344.73, 34.47-310.26, 34.47-275.79, 34.47-241.31, 34.47-206.84, 34.47-172.37, 34.47-137.89, 34.47-103.42, 34.47-68.95, 68.95-344.73, 68.95-310.26, 68.95-275.79, 68.95-241.31, 68.95-206.84, 68.95-172.37, 68.95-137.89, 68.95-103.42, 103.42-344.73, 103.42-310.26, 103.42-275.79, 103.42-241.31, 103.42-206.84, 103.42-172.37, 103.42-137.89, 137.89-344.73, 137.89-310.26, 137.89-275.79, 137.89-241.31, 137.89-206.84, 137.89-172.37, 172.37-344.73, 172.37-310.26, 172.37-275.79, 172.37-241.31, 172.37-206.84, 206.84-344.73, 206.84-310.26, 206.84-275.79, 206.84-241.31, 275.79-344.73, 275.79-310.26, 310.26-344.73 bar (100-5,000, 100-4,500, 100-4,000, 100-3,500, 100-3,000, 100-2,500, 100-2,000, 100-1,500, 100-1,000, 100-500, 500-5,000, 500-4,500, 500-4,000, 500-3,500, 500-3,000, 500-2,500, 500-2,000, 500-1,500, 500-1,000, 1,000-5,000, 1,000-4,500, 1,000-4,000, 1,000-3,500, 1,000-3,000, 1,000-2,500, 1,000-2,000, 1,000-1,500, 1,500-5,000, 1,500-4,500, 1,500-4,000, 1,500-3,500, 1,500-3,000, 1,500-2,500, 1,500-2,000, 2,000-5,000, 2,000-4,500, 2,000-4,000, 2,000-3,500, 2,000-3,000, 2,000-2,500, 2,500-5,000, 2,500-4,500, 2,500-4,000, 2,500-3,500, 2,500-3,000, 3,000-5,000, 3,000-4,500, 3,000-4,000, 3,000-3,500, 4,000-5,000, 4,000-4,500 or 4,500-5,000 psi), no more than 344.73, 310.26, 275.79, 241.31, 206.84, 172.37, 137.89, 103.42, 68.95 or 34.47 bar (5,000, 4,500, 4,000, 3,500, 3,000, 2,5000, 2,000, 1,500, 1,000 or 500 psi), or at least 310.26, 275.79, 241.31, 206.84, 172.37, 137.89, 103.42, 68.95 or 34.47 bar (4,500, 4,000, 3,500, 3,000, 2,5000, 2,000, 1,500, 1,000 or 500 psi).

The maximum extrusion force for pushing the tissue graft from the cannula to the treatment site may be 100-300, 100-250, 100-200, 100-190, 100-180, 110-170, 110-160, 100-150, 100-140, 100-130, 100-120, 100-110, 110-300, 110-250, 110-200, 110-190, 110-180, 110-170, 110-160, 110-150, 110-140, 110-130, 110-120, 120-300, 120-250, 120-200, 120-190, 120-180, 120-170, 120-160, 120-150, 120-140, 120-130, 130-300, 130-250, 130-200, 130-190, 130-180, 130-170, 130-160, 130-150, 130-140, 140-300, 140-250, 140-200, 140-190, 140-180, 140-170, 140-160, 140-150, 150-300, 150-250, 150-200, 150-190, 150-180, 150-170, 150-160, 160-300, 160-250, 160-200, 160-190, 160-180, 160-170, 170-300, 170-250, 170-200, 170-190, 170-180, 180-300, 180-250, 180-200, 180-190, 190-300, 190-250, 190-200, 200-300, 200-250 or 250-300 N, no more than 110, 120, 130, 140, 150, 160, 170, 180, 190, 200, 250 or 300 N, or no less than 100, 110, 120, 130, 140, 150, 160, 170, 180, 190, 200, 250 or 300 N. In one embodiment, the maximum extrusion force may be 180-190 N (e.g., 187 N) or 150-160 N (e.g., 152 N).

For each cannula of the present invention, methods for preparing the cannulas are provided. In certain aspects, preparation methods comprise preparing a tissue plug by loading and pressing tissue graft in a loading chamber. In one aspect, the method further comprises placing a tissue backstop into the bore from the proximal end of the cannula. The tissue backstop occupies a portion of the bore and a void is formed by the remaining portion of the bore. The preparation method further comprises loading a first tissue plug into the void from the distal end of the cannula, wherein the first tissue plug may have a sectional area smaller than the sectional area of the inner diameter of the cannula; pushing the first tissue plug towards the proximal end of the void such that the first tissue graft is in contact with the tissue backstop. In another aspect, a second tissue plug is loaded into the void from the distal end, and the second tissue plug may have a sectional area smaller than the sectional area of the inner diameter of the cannula; pushing the second tissue plug towards the proximal end of the void such that the second tissue plug is in contact with the first tissue plug. The first tissue plug and the second tissue plug together may form a tissue graft in the bore.

Another method for preparing the cannula of the present invention is further provided. The preparation method comprises placing a tissue backstop into the bore from the proximal end of the cannula such that the tissue backstop occupies a portion of the bore and a void is formed by the remaining portion of the bore. The preparation method also comprises loading a first tissue plug into the void from the distal end of the cannula, and the first tissue plug has a cross-sectional diameter smaller than the inner diameter of the cannula. The preparation method further comprises pushing the first tissue plug towards the proximal end of the void such that the first tissue graft is in contact with the tissue backstop. The preparation method further comprises loading a second tissue plug into the void from the distal end, and the second tissue plug has a cross-sectional diameter smaller than the inner diameter of the cannula. The preparation method further comprises pushing the second tissue plug towards the proximal end of the void such that the second tissue graft is in contact with the first tissue plug. The first tissue plug and the second tissue plug form the tissue graft in the cannula.

The predetermined final loading pressure according to the invention may be about 0.07-68.95, 0.69-68.95, 1.38-68.95, 3.45-68.95, 6.89-68.95, 0.07-34.47, 0.69-34.47, 1.38-34.47, 3.45-34.47, 6.89-34.47, 0.07-27.58, 0.69-27.58, 1.38-27.58, 3.45-27.58, 6.89-27.58, 0.07-20.68, 0.69-20.68, 1.38-20.68, 3.45-20.68, 6.89-20.68, 0.07-13.79, 0.69-13.79, 1.38-13.79, 2.07-13.79, 2.76-13.79, 3.45-13.79, 4.14-13.79, 4.83-13.79, 5.52-13.79, 6.21-13.79, 6.89-13.79, 7.58-13.79, 8.27-13.79, 8.96-13.79, 9.65-13.79, 10.34-13.79, 1.38-10.34, 2.07-10.34, 2.76-10.34, 3.45-10.34, 4.14-10.34, 4.83-10.34, 5.52-10.34, 6.21-10.34, 6.89-10.34, 7.58-10.34, 8.27-10.34, 8.96-10.34, 9.65-10.34, 1.38-9.65, 2.07-9.65, 2.76-9.65, 3.45-9.65, 4.14-9.65, 4.83-9.65, 5.52-9.65, 6.21-9.65, 6.89-9.65, 7.58-9.65, 8.27-9.65, 8.96-9.65, 1.38-8.96, 2.07-8.96, 2.76-8.96, 3.45-8.96, 4.14-8.96, 4.83-8.96, 5.52-8.96, 6.21-8.96, 6.89-8.96, 7.58-8.96, 8.27-8.96, 1.38-8.27, 2.07-8.27, 2.76-8.27, 3.45-8.27, 4.14-8.27, 4.83-8.27, 5.52-8.27, 6.21-8.27, 6.89-8.27, 7.58-8.27, 1.38-7.58, 2.07-7.58, 2.76-7.58, 3.45-7.58, 4.14-7.58, 4.83-7.58, 5.52-7.58, 6.21-7.58, 6.89-7.58, 1.38-6.89, 2.07-6.89, 2.76-6.89, 3.45-6.89, 4.14-6.89, 4.83-6.89, 5.52-6.89, 6.21-6.89, 1.38-6.21, 2.07-6.21, 2.76-6.21, 3.45-6.21, 4.14-6.21, 4.83-6.21, 5.52-6.21, 1.38-5.52, 2.07-5.52, 2.76-5.52, 3.45-5.52, 4.14-5.52, 4.83-5.52, 1.38-4.83, 2.07-4.83, 2.76-4.83, 3.45-4.83, 4.14-4.83, 1.38-4.14, 2.07-4.14, 2.76-4.14, 3.45-4.14, 1.38-3.45, 2.07-3.45, 2.76-3.45, 1.38-2.76 or 2.07-2.76 bar (1-1,000, 10-1,000, 20-1,000, 50-1,000, 100-1,000, 1-500, 10-500, 20-500, 50-500, 100-500, 1-400, 10-400, 20-400, 50-400, 100-400, 1-300, 10-300, 20-300, 50-300, 100-300, 1-200, 10-200, 20-200, 30-200, 40-200, 50-200, 60-200, 70-200, 80-200, 90-200, 100-200, 110-200, 120-200, 130-200, 140-200, 150-200, 20-150, 30-150, 40-150, 50-150, 60-150, 70-150, 80-150, 90-150, 100-150, 110-150, 120-150, 130-150, 140-150, 20-140, 30-140, 40-140, 50-140, 60-140, 70-140, 80-140, 90-140, 100-140, 110-140, 120-140, 130-140, 20-130, 30-130, 40-130, 50-130, 60-130, 70-130, 80-130, 90-130, 100-130, 110-130, 120-130, 20-120, 30-120, 40-120, 50-120, 60-120, 70-120, 80-120, 90-120, 100-120, 110-120, 20-110, 30-110, 40-110, 50-110, 60-110, 70-110, 80-110, 90-110, 100-110, 20-100, 30-100, 40-100, 50-100, 60-100, 70-100, 80-100, 90-100, 20-90, 30-90, 40-90, 50-90, 60-90, 70-90, 80-90, 20-80, 30-80, 40-80, 50-80, 60-80, 70-80, 20-70, 30-70, 40-70, 50-70, 60-70, 20-60, 30-60, 40-60, 50-60, 20-50, 30-50, 40-50, 20-40 or 30-40 psi). For example, the predetermined final loading pressure may be 4,34-7,65 bar (63-111 psi).

The preparation method may further comprise, for example, loading a third tissue plug into the void from the distal end of the cannula, wherein the third tissue plug may have a sectional area smaller than the inner diameter of the cannula; and pushing the third tissue plug towards the distal end such that the third tissue plug is in contact with the second tissue plug. The first tissue plug, the second tissue plug and the third tissue plug may form a tissue graft in the opening space. The predetermined final loading pressure may be about 0.07-68.95, 0.69-68.95, 1.38-68.95, 3.45-68.95, 6.89-68.95, 0.07-34.47, 0.69-34.47, 1.38-34.47, 3.45-34.47, 6.89-34.47, 0.07-27.58, 0.69-27.58, 1.38-27.58, 3.45-27.58, 6.89-27.58, 0.07-20.68, 0.69-20.68, 1.38-20.68, 3.45-20.68, 6.89-20.68, 0.07-13.79, 0.69-13.79, 1.38-13.79, 2.07-13.79, 2.76-13.79, 3.45-13.79, 4.14-13.79, 4.83-13.79, 5.52-13.79, 6.21-13.79, 6.89-13.79, 7.58-13.79, 8.27-13.79, 8.96-13.79, 9.65-13.79, 10.34-13.79, 1.38-10.34, 2.07-10.34, 2.76-10.34, 3.45-10.34, 4.14-10.34, 4.83-10.34, 5.52-10.34, 6.21-10.34, 6.89-10.34, 7.58-10.34, 8.27-10.34, 8.96-10.34, 9.65-10.34, 1.38-9.65, 2.07-9.65, 2.76-9.65, 3.45-9.65, 4.14-9.65, 4.83-9.65, 5.52-9.65, 6.21-9.65, 6.89-9.65, 7.58-9.65, 8.27-9.65, 8.96-9.65, 1.38-8.96, 2.07-8.96, 2.76-8.96, 3.45-8.96, 4.14-8.96, 4.83-8.96, 5.52-8.96, 6.21-8.96, 6.89-8.96, 7.58-8.96, 8.27-8.96, 1.38-8.27, 2.07-8.27, 2.76-8.27, 3.45-8.27, 4.14-8.27, 4.83-8.27, 5.52-8.27, 6.21-8.27, 6.89-8.27, 7.58-8.27, 1.38-7.58, 2.07-7.58, 2.76-7.58, 3.45-7.58, 4.14-7.58, 4.83-7.58, 5.52-7.58, 6.21-7.58, 6.89-7.58, 1.38-6.89, 2.07-6.89, 2.76-6.89, 3.45-6.89, 4.14-6.89, 4.83-6.89, 5.52-6.89, 6.21-6.89, 1.38-6.21, 2.07-6.21, 2.76-6.21, 3.45-6.21, 4.14-6.21, 4.83-6.21, 5.52-6.21, 1.38-5.52, 2.07-5.52, 2.76-5.52, 3.45-5.52, 4.14-5.52, 4.83-5.52, 1.38-4.83, 2.07-4.83, 2.76-4.83, 3.45-4.83, 4.14-4.83, 1.38-4.14, 2.07-4.14, 2.76-4.14, 3.45-4.14, 1.38-3.45, 2.07-3.45, 2.76-3.45, 1.38-2.76 or 2.07-2.76 bar (1-1,000, 10-1,000, 20-1,000, 50-1,000, 100-1,000, 1-500, 10-500, 20-500, 50-500, 100-500, 1-400, 10-400, 20-400, 50-400, 100-400, 1-300, 10-300, 20-300, 50-300, 100-300, 1-200, 10-200, 20-200, 30-200, 40-200, 50-200, 60-200, 70-200, 80-200, 90-200, 100-200, 110-200, 120-200, 130-200, 140-200, 150-200, 20-150, 30-150, 40-150, 50-150, 60-150, 70-150, 80-150, 90-150, 100-150, 110-150, 120-150, 130-150, 140-150, 20-140, 30-140, 40-140, 50-140, 60-140, 70-140, 80-140, 90-140, 100-140, 110-140, 120-140, 130-140, 20-130, 30-130, 40-130, 50-130, 60-130, 70-130, 80-130, 90-130, 100-130, 110-130, 120-130, 20-120, 30-120, 40-120, 50-120, 60-120, 70-120, 80-120, 90-120, 100-120, 110-120, 20-110, 30-110, 40-110, 50-110, 60-110, 70-110, 80-110, 90-110, 100-110, 20-100, 30-100, 40-100, 50-100, 60-100, 70-100, 80-100, 90-100, 20-90, 30-90, 40-90, 50-90, 60-90, 70-90, 80-90, 20-80, 30-80, 40-80, 50-80, 60-80, 70-80, 20-70, 30-70, 40-70, 50-70, 60-70, 20-60, 30-60, 40-60, 50-60, 20-50, 30-50, 40-50, 20-40 or 30-40 psi). For example, the predetermined final loading pressure may be 4,34-7,65 bar (63-111 psi).

It is understood that any number of tissue plugs may be employed to load a cannula, for example, one, two, three, four or more tissue plugs, according to the principles of the invention.

The preparation method may further comprise preparing the first tissue plug, and/or the second tissue plug and/or the third tissue plug, for example, in a predetermined shape. The predetermined shape may be a cylinder suitable for loading into a cannula. In one embodiment, the predetermined shape may have a diameter smaller than the inner diameter of the cannula for ease of loading and to avoid potential jamming caused by cannula surface roughness, misalignment, tissue plug expansion, and the like.

The tissue plug may be prepared in a loading device according to the principles of the invention. The loading device may have a chamber and the chamber may be cylindrical. The cylindrical chamber may have a cross-sectional diameter smaller than the inner diameter of the cannula by, for example, 0.1-1, 0.1-0.5, 0.2-1, 0.2-0.5, 0.3-1, 0.3-0.5, 0.4-1, 0.4-0.5 or 0.5-1 mm. The preparation method may further comprise pressing the first tissue into a first tissue plug in the cylindrical chamber. The preparation method may further comprise pressing the second tissue into a second tissue plug in the cylindrical chamber. The preparation method may further comprise pressing additional tissue into subsequent tissue plugs in the cylindrical chamber.

The loading device may comprise a first component and a second component. The first component has a first opening. The second component has a second opening. The chamber in the loading device may formed by mating the first opening and the second opening to form a chamber. The chamber may have a cross-section, optionally a circular cross-section. In certain two component embodiments, each component contributes substantially equally to the overall cross-section of the tissue plug and, in other designs, one component forms a greater portion of the cross-section such that the first opening constitutes 50-80%, 50-70% or 50-60%, at least 50%, 60%, 70% or 80%, or less than 50%, 60%, 70% or 80% of the circular cross-section of the chamber. It is understood that multiple components, such as three or more, may be constructed to create a chamber in the loading device that may be used to form a tissue plug suitable for loading into a cannula according to the invention.

The inner surface of the chamber may have surface roughness of 0.508-1.27, 0.508-1.016, 0.508-0.762, 0.762-1.27, 0.762-1.016, 1.016-1.27, no more than 0.508, 0.762, 0.889, 1.016, 1.27, or nor less than 0.508, 0.762, 1.016, 1.27 micrometer (20-50, 20-40, 20-30, 30-50, 30-40, 40-50, no more than 20, 30, 35, 40 or 50, or no less than 20, 30, 40 of 50 microinches) Root Mean Square (RMS). In one embodiment, the inner surface of the chamber may have surface roughness of no more than 0.81 micrometer RMS (32 microinches RMS).

The interior surface of the cannula may have surface roughness of 0.508-1.27, 0.508-1.016, 0.508-0.762, 0.762-1.27, 0.762-1.016, 1.016-1.27, no more than 0.508, 0.762, 0.889, 1.016, 1.27, or nor less than 0.508, 0.762, 1.016, 1.27 micrometer (20-50, 20-40, 20-30, 30-50, 30-40, 40-50, no more than 20, 30, 35, 40 or 50, or no less than 20, 30, 40 of 50 microinches) RMS. In one embodiment, the interior surface of the cannula has surface roughness of no more than 0.81 micrometer RMS (32 microinches RMS).

The term "about" as used herein when referring to a measurable value such as an amount, a percentage, and the like, is meant to encompass variations of ±20% or ±10%, ±5%, ±1%, or ±0.1% from the specified value, as such variations are appropriate.

### Example 1. Loading

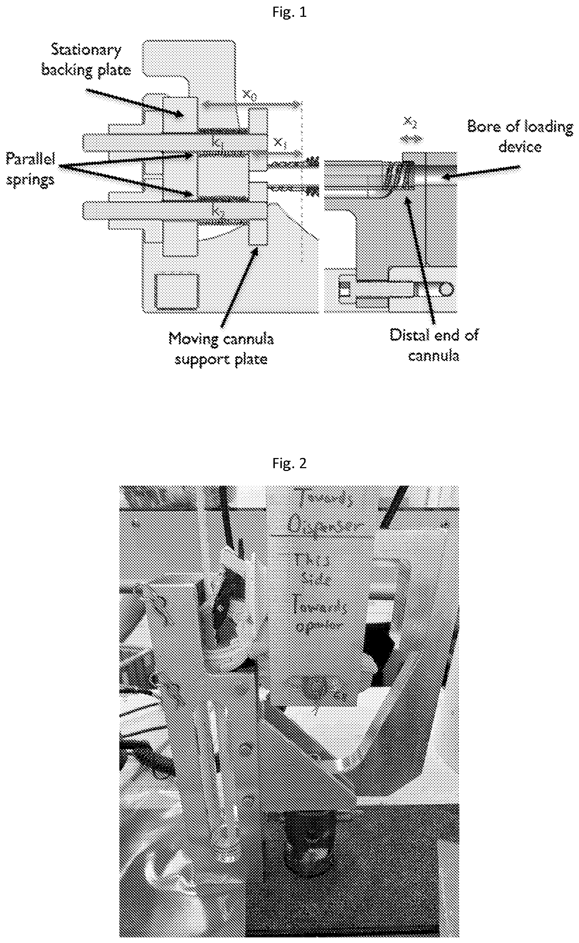

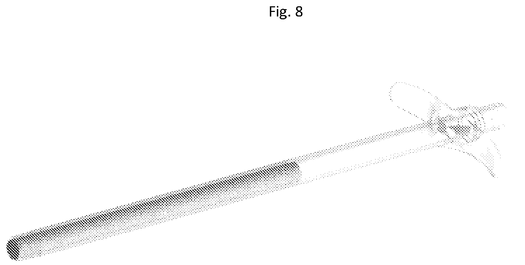

Testing was performed on a loading device as depicted in Fig. 1 using spring resistance (k) and a visual indicator of spring compression distance (x) to provide visual feedback on final compression force (F = k*x) and hence final loading pressure (P = F/A). The figure shows a moving cannula support plate with a dowel pressed into it which slides into the cannula bore from the proximal end, until the proximal end of the cannula comes in contact with the moving cannula support plate. The dowel was employed, through a portion of the cannula, to provide support for the cannula and to define the void to be filled by tissue graft. The backing plate and cannula were pressed forward by springs that were compressed between the moving cannula support plate and a stationary backing plate. The distal end of the cannula was fixed into a 0.635 mm (0.25in) deep counterbore leading to the loading chamber of the loading device. The loading device was designed with two springs in parallel, so the effective spring coefficient is the sum of the two individual spring coefficients. The effective spring coefficient is 1.166 N/m (6.66lb/in). The uncompressed spring length was 25,4 mm (1in). When the cannula was loaded into the loading device, the springs were compressed by 11.18 mm (0.44in), creating a compression force on the cannula at (k₁+k₂)*x₁, that is (583+583)*0,01118m = 13.03 N ((3.33+3.33)*(.44), i.e., 6.66lb/in *.44in = 2.93lb). This compression force prevented the cannula from moving toward the springs and away from the tissue graft being loaded at the distal end of the cannula. Once a tissue plug entered the cannula and was pushed to the back of the void, the slowly increasing loading force was transmitted through the tissue plug into the dowel until the calculated 13.03N (2.93lb) of spring compression force was overcome, causing the cannula to start to move out of the counterbore towards the proximal end of the cannula. If the cannula moves completely out of the counterbore, the springs will be compressed at least another 0.635mm (0.25in), meaning the cannula removal force on the tissue has risen to at least (k₁+k₂)*(x₁+x₂), that is 1.166 N/m * 17.52mm = 20,428 N ((3.33+3.33)*(.44+.25), i.e., 6.66lb/in *(.44in + .25in) = 4.60lb). If the cannula moves, but does not leave the counterbore, the compression force on the tissue will remain between these two limits. Since the cannula ID is 6mm (0.23622in), its cross-sectional area is 28,274137 mm² (0.043825in²). The final loading pressure limits are then 4.61 bar (2.93lb / 0.043825in² = 66.9psi) and 7.23 bar (4.60lb / 0.043825in² = 104.9psi), respectively. The resulting tissue graft loaded into the cannula is depicted in Fig. 8.

### Example 2. Tissue graft extrusion testing

A study was performed to provide evidence that the loading tool is capable of consistent and reliable filling of the cannula with a tissue graft to demonstrate the ability of the loading tool to overcome donor and operator variation in controlling the force to extrude cannula from the delivery device. The resulting tissue graft loaded in the cannula were extruded from the cannula using a delivery device. The extrusion of the tissue graft from the cannula using a delivery device was experimentally tested in a laboratory and the force was measured using mechanical testing techniques. The results of the study confirmed that the force to extrude the tissue graft from the cannula using a delivery device was suitable because it was less than the acceptance criteria determined through ergonomics and human factors research for the intended use by the intended user (e.g., surgeon) in a clinical setting.

Cannulas loaded with the aliquot tool were extruded using a delivery device placed in a test fixture, where motion and forces were controlled by an Instron testing machine. The delivery device was held with the distal end with the plunger facing down, while the Instron pulled up on the handle of the delivery device, as seen in Fig. 2, and measured the resistance force from this handle during extrusion using a load cell connected to a computer.

The study design included tissue graft filled cannulas prepared from four different tissue donors, mixed with cryoprotectant and filled using the loading tool to a final volume of 3 cm³ (cc). Multiple replicates were made of each tissue graft filled cannula sample for testing by four different operators. Tissue graft filled cannula were packaged, cryopreserved and transferred to frozen storage for 48 hrs prior to testing. Tissue graft filled cannula were thawed using a water bath and coupled with the delivery system to prepare for extrusion.

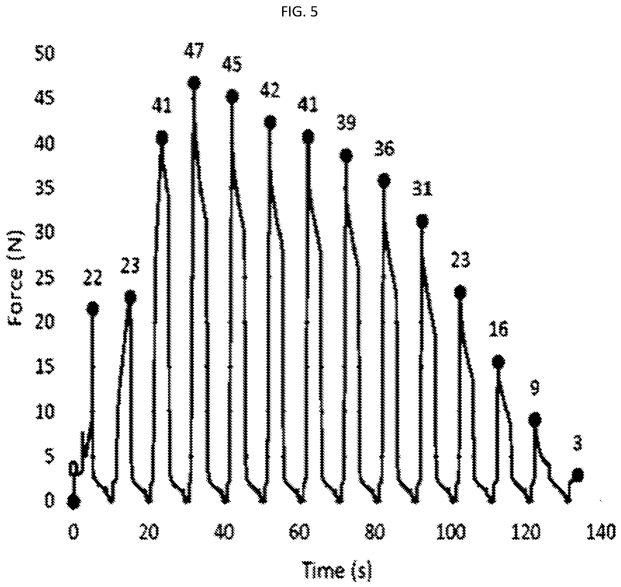

An example of typical force versus time data obtained in this study is shown in Fig. 5. When the test starts, the computer starts collecting, at a high sampling rate, the values from the load cell reading the resistance of the delivery device handle actuation. The push rod has already been advanced though the empty part of the cannula so that it is adjacent to the tissue graft. Each time the handle is actuated, a stroke cycle occurs where the push rod advances about 9 mm or a volume of about 0.25 cm³. At the beginning of the test, the push rod is just coming into contact with the tissue. Since the tissue graft has more surface area in contact with the cannula walls than the individual tissue plugs that were loaded, and hence more friction, it is believed that the tissue compresses a small amount before the tissue at the distal end begins to extrude out of the cannula. The force required to extrude increases as the tissue compresses or becomes more tightly packed with further strokes. Then at some point it is theorized that the decreasing surface area due to push rod advancement and tissue extrusion leads to a decrease in the extrusion force measured until the cannula is empty. Once the cannula is empty, the force reading will only be a function of friction and mass of the delivery device and fixture.

To analyze the maximum extrusion forces, only the peak force from each stroke is needed. This experimental setup measures the force on the delivery device handle, of which the maximum values from each cannula are shown in Table 1. It is also of interest to estimate the force being directly applied to the tissue from the push rod. An estimate of the mechanical advantage of the delivery device in the test fixture was made using measurements of the geometry and found to be approximately 3.8. This mechanical advantage was multiplied by the peak force data from each stroke and plotted for several cannula in Fig. 6. To analyze the effect of the translation distance and compression of the tissue graft on extrusion force, some estimates of tissue surface contact with the cannula were calculated. The last stroke recorded, which has very little resistance force, is assumed to be empty and so the stroke prior to that has approximately 0.25 cm³ tissue based on the spacing of teeth on the push rod in the delivery device. Working backwards from the last stroke, the length of the graft in the cannula was determined and therefore the surface area in contact with the cannula at each stroke was estimated. The peak force from Fig. 6 was then divided by the assumed surface area of contact between the graft and cannula, plotted in Fig. 7, to show that the friction force is likely increasing, possibly due to graft compression and increased force on the cannula walls, even as tissue is being extruded from the cannula.

The results indicated that all samples had a peak extrusion force less than 187N (42 lbf). The average extrusion force was 74N (16.6 lbf) with a standard deviation of 23N (5.17lbf). The actuation method used in the engineering study in combination with the geometry of the delivery device had a conservatively approximated mechanical advantage of 3.8, which would lead to an average of force applied directly to the tissue of 281.2N with a standard deviation of 87.4N. The study concluded that there is 95% confidence that 95% of the observed population of extrusion forces with the tissue filled cannula delivery system will be 154N or less, below the acceptance criteria of 187N. As shown in Table 1, the results of this study demonstrate that aliquot tool has been validated for its intended use to control loading forces when aliquoting tissue graft in the cannula.

The tissue weight in each ranged from 3.5g to 3.8g, and the tissue density was from 1.1 g/cm³ (3.5g/3cm³) to 1.3 g/cm³ (3.8g/3cm³).

Results of this study demonstrate that the aliquot tool has been validated for its intended use to control loading forces when loading graft tissue into the cannula.

Other embodiments of the invention will be apparent to those skilled in the art from consideration of the specification and practice of the invention disclosed herein. It is intended that the specification and examples be considered as exemplary only, with the true scope of the invention being defined by the following claims.

**Table 1. Mechanical Extrusion Results**

| Donor | Sample Number | Operator & Maximum Extrusion Force (N) | | | |
|---|---|---|---|---|---|
| | | 1 | 2 | 3 | 4 |
| 1914254 | 2 | 61 | 50 | 73 | 64 |
| | 3 | 83 | 69 | 96 | 68 |
| | 4 | 89 | 22 | 44 | 73 |
| | 5 | 85 | 76 | 70 | 56 |
| 2010300 | 2 | 101 | 108 | 63 | 81 |
| | 3 | 108 | 122 | 35 | 90 |
| | 4 | 84 | 122 | 37 | 57 |
| | 5 | 63 | 121 | 102 | 81 |
| 2011087 | 2 | 94 | 54 | 62 | 81 |
| | 3 | 58 | 51 | 73 | 77 |
| | 4 | 121 | 84 | 56 | 68 |
| | 5 | 115 | 54 | 59 | 82 |
| 2013206 | 2 | 87 | 67 | 49 | 84 |
| | 3 | 78 | 59 | 51 | 63 |
| | 4 | 60 | 32 | 67 | 106 |
| | 5 | 74 | 44 | 43 | 101 |
| 95% confidence that 95% of the population is below | | 134 | 152 | 109 | 113 |
| Average | 74 | | | | |
| StDev | 23 | | | | |

## Claims

1. A cannula for delivering a tissue graft to a treatment site, comprising a distal end, a proximal end, a bore formed by an interior surface of the cannula, extending from the distal end to the proximal end and having an inner diameter, wherein the cannula is configured to hold a tissue graft inside the bore and in contact with the interior surface, wherein the cannula is further configured to release the tissue graft from the cannula at the distal end to the treatment site, wherein the tissue graft has a final loading pressure of 1,38 - 13,8 bar (20-200 psi) and wherein at least one of conditions i), ii), iii), and/or iv) are met:
i) the tissue graft has a maximum extrusion force of less than 190 N;
ii) the cannula further comprises more than 5000 viable bone cells or bone forming cells per cm³, wherein the tissue graft is a bone graft;
iii) the cannula further comprises a cryopreservation solution in the bore, wherein the tissue graft is a bone graft comprising bone particulates having viable bone cells or bone forming cells, and the volume ratio of the cryopreservation solution to the bone graft is less than 2;
iv) the cannula further comprises a cryopreservation solution in the bore, wherein the tissue graft is a bone graft, the bone graft comprises bone particulates having viable bone cells or bone forming cells, and the bone graft excludes demineralized bone matrix.

2. The cannula of claim 1, wherein the cannula has a particulate density of 1.1-1.3 g/cm³.

3. The cannula of claim 1, wherein the tissue graft is a cryopreserved bone graft, the bone graft comprises demineralized ground cortical bone and nondemineralized bone chips, the bone chips comprise cortical, corticocancellous, cancellous, or combinations thereof, and the bone chips comprise viable bone-forming cells.

4. A system for delivering a tissue graft to a treatment site, comprising:
(a) the cannula of any one of claims 1-3; and
(b) a delivery device comprising a push rod and a push handler, wherein the push rod is in contact with the tissue graft and on the opposite side of the tissue graft from the distal end, and wherein the push handler forces the push rod to push the tissue graft out of the cannula at the distal end to the treatment site.

5. A method for preparing the cannula of any one of claims 1-3 delivering a tissue graft to a treatment site, comprising
(a) connecting the cannula with a delivery device, wherein the delivery device comprises a push rod;
(b) contacting the tissue graft with the push rod, wherein the push rod is on the opposite side of the tissue graft from the distal end.

6. The method of claim 5, wherein the push rod is configured to push the tissue graft at a pressure of no more than 275,79 bar (4000 psi) following step (b).

7. A method for preparing the cannula of any one of claims 1-3 for delivering a tissue graft to a treatment site, comprising
(a) placing a tissue backstop into the bore from the proximal end of the cannula, whereby the tissue backstop occupies a portion of the bore and a void is formed by the remaining portion of the bore;
(b) loading a first tissue plug into the void from the distal end of the cannula, wherein the first tissue plug has a cross-sectional diameter smaller than the inner diameter of the cannula; and
(c) pushing the first tissue plug towards the proximal end of the void, whereby the first tissue graft is in contact with the tissue backstop.

8. The method of claim 7, further comprising
(d) loading a second tissue plug into the void from the distal end, wherein the second tissue plug has a cross-sectional diameter smaller than the inner diameter of the cannula; and
(e) pushing the second tissue plug towards the proximal end of the void, whereby the second tissue graft is in contact with the first tissue plug, whereby the first tissue plug and the second tissue plug form the tissue graft in the cannula.

9. The method of claim 8, further comprising:
(f) loading a third tissue plug into the void from the distal end, wherein the third tissue plug has a cross-sectional diameter smaller than the inner diameter of the cannula; and
(g) pushing the third tissue plug towards the proximal end of the void, whereby the third tissue plug is in contact with the second tissue plug, and the first tissue plug, the second tissue plug and the third tissue plug form the tissue graft in the cannula.

10. The method of claim 9, further comprising preparing each of the first tissue plug and the second tissue plug in a cylindrical chamber of a loading device, wherein the chamber has a surface finish of no more than 0,81 micrometers (32 microinches) root mean square.

## Patentansprüche

1. Kanüle zum Einbringen eines Gewebetransplantats an eine Behandlungsstelle, umfassend ein distales Ende, ein proximales Ende, eine durch eine Innenfläche der Kanüle gebildete Bohrung, die sich vom distalen Ende zum proximalen Ende erstreckt und einen Innendurchmesser aufweist, wobei die Kanüle so ausgebildet ist, dass sie ein Gewebetransplantat innerhalb der Bohrung und in Kontakt mit der Innenfläche hält, wobei die Kanüle ferner so ausgebildet ist, dass sie das Gewebetransplantat am distalen Ende aus der Kanüle an die Behandlungsstelle abgibt, wobei das Gewebetransplantat einen Endladedruck von 1,38 - 13,8 bar (20-200 psi) aufweist und wobei mindestens eine der Bedingungen i), ii), iii) und/oder iv) erfüllt ist:
i) das Gewebetransplantat weist eine maximale Extrusionskraft von weniger als 190 N auf;
ii) die Kanüle umfasst ferner mehr als 5000 lebensfähige Knochenzellen oder knochenbildende Zellen pro cm³, wobei es sich bei dem Gewebetransplantat um ein Knochentransplantat handelt;
iii) die Kanüle enthält ferner eine Kryokonservierungslösung in der Bohrung, wobei das Gewebetransplantat ein Knochentransplantat ist, das Knochenpartikel mit lebensfähigen Knochenzellen oder knochenbildenden Zellen umfasst, und das Volumenverhältnis der Kryokonservierungslösung zum Knochentransplantat kleiner als 2 ist;
iv) die Kanüle umfasst ferner eine Kryokonservierungslösung in der Bohrung, wobei das Gewebetransplantat ein Knochentransplantat ist, das Knochentransplantat Knochenpartikel mit lebensfähigen Knochenzellen oder knochenbildenden Zellen umfasst und das Knochentransplantat keine demineralisierte Knochenmatrix enthält.

2. Kanüle nach Anspruch 1, wobei die Kanüle eine Partikeldichte von 1,1-1,3 g/cm³ aufweist.

3. Kanüle nach Anspruch 1, wobei das Gewebetransplantat ein kryokonserviertes Knochentransplantat ist, das Knochentransplantat aus demineralisiertem, gemahlenem Kortikalknochen und nicht demineralisierten Knochensplittern besteht, die Knochensplitter Kortikal-, Kortikospongiosa-, Spongiosa- oder Kombinationen davon umfassen und die Knochensplitter lebensfähige knochenbildende Zellen enthalten.

4. System zum Einbringen eines Gewebetransplantats an eine Behandlungsstelle, umfassend:
(a) die Kanüle nach einem der Ansprüche 1-3; und
(b) eine Zuführvorrichtung, die eine Schubstange und einen Schubgriff umfasst, wobei die Schubstange mit dem Gewebetransplantat in Kontakt steht und sich auf der dem distalen Ende gegenüberliegenden Seite des Gewebetransplantats befindet, und wobei der Schubgriff die Schubstange dazu zwingt, das Gewebetransplantat am distalen Ende aus der Kanüle zur Behandlungsstelle zu schieben.

5. Verfahren zur Vorbereitung der Kanüle nach einem der Ansprüche 1-3 zum Zuführen eines Gewebetransplantats zu einer Behandlungsstelle, umfassend
(a) das Verbinden der Kanüle mit einer Zuführvorrichtung, wobei die Zuführvorrichtung eine Schubstange umfasst;
(b) das Inkontaktbringen des Gewebetransplantats mit der Schubstange, wobei sich die Schubstange auf der dem distalen Ende gegenüberliegenden Seite des Gewebetransplantats befindet.

6. Verfahren nach Anspruch 5, wobei die Schubstange so ausgelegt ist, dass sie das Gewebetransplantat nach Schritt (b) mit einem Druck von nicht mehr als 275,79 bar (4000 psi) schiebt.

7. Verfahren zur Vorbereitung der Kanüle nach einem der Ansprüche 1 bis 3 zur Abgabe eines Gewebetransplantats an eine Behandlungsstelle, umfassend
(a) das Einbringen eines Gewebeanschlags in die Bohrung vom proximalen Ende der Kanüle aus, wobei der Gewebeanschlag einen Teil der Bohrung einnimmt und durch den verbleibenden Teil der Bohrung ein Hohlraum gebildet wird;
(b) das Einführen eines ersten Gewebestopfens in den Hohlraum vom distalen Ende der Kanüle aus, wobei der erste Gewebestopfen einen Querschnittsdurchmesser aufweist, der kleiner ist als der Innendurchmesser der Kanüle; und
(c) das Verschieben des ersten Gewebestopfens in Richtung des proximalen Endes des Hohlraums, wodurch das erste Gewebetransplantat mit dem Gewebestopper in Kontakt steht.

8. Verfahren nach Anspruch 7, ferner umfassend:
(d) das Einführen eines zweiten Gewebestopfens in den Hohlraum vom distalen Ende her, wobei der zweite Gewebestopfen einen Querschnittsdurchmesser aufweist, der kleiner ist als der Innendurchmesser der Kanüle; und
(e) das Verschieben des zweiten Gewebestopfen in Richtung des proximalen Endes des Hohlraums, wodurch das zweite Gewebetransplantat mit dem ersten Gewebestopfen in Kontakt kommt, wodurch der erste Gewebestopfen und der zweite Gewebestopfen das Gewebetransplantat in der Kanüle bilden.

9. Verfahren nach Anspruch 8, das ferner umfasst:
(f) Einführen eines dritten Gewebestopfens in den Hohlraum vom distalen Ende her, wobei der dritte Gewebestopfen einen Querschnittsdurchmesser aufweist, der kleiner ist als der Innendurchmesser der Kanüle; und
(g) Vorschieben des dritten Gewebestopfens in Richtung des proximalen Endes des Hohlraums, wodurch der dritte Gewebestopfen mit dem zweiten Gewebestopfen in Kontakt kommt und der erste Gewebestopfen, der zweite Gewebestopfen und der dritte Gewebestopfen das Gewebetransplantat in der Kanüle bilden.

10. Verfahren nach Anspruch 9, ferner umfassend das Vorbereiten sowohl des ersten Gewebestopfens als auch des zweiten Gewebestopfens in einer zylindrischen Kammer einer Ladevorrichtung, wobei die Kammer eine Oberflächenrauheit von nicht mehr als 0,81 Mikrometer (32 Mikroinch) im quadratischen Mittelwert aufweist.

## Revendications

1. Canule destinée à acheminer une greffe tissulaire vers un site de traitement, comprenant une extrémité distale, une extrémité proximale, un alésage formé par une surface intérieure de la canule, s'étendant de l'extrémité distale à l'extrémité proximale et présentant un diamètre intérieur, dans laquelle la canule est configurée pour maintenir une greffe tissulaire à l'intérieur de l'alésage et en contact avec la surface intérieure, la canule étant en outre configurée pour libérer le greffon tissulaire de la canule au niveau de l'extrémité distale vers le site de traitement, le greffon tissulaire présentant une pression de chargement finale comprise entre 1,38 et 13,8 bars (20-200 psi) et au moins l'une des conditions i), ii), iii) et/ou iv) étant remplie :
i) le greffon tissulaire présente une force d'extrusion maximale inférieure à 190 N ;
ii) la canule comprend en outre plus de 5 000 cellules osseuses viables ou cellules ostéogéniques par cm³, le greffon tissulaire étant un greffon osseux ;
iii) la canule comprend en outre une solution de cryoconservation dans son alésage, dans laquelle le greffon tissulaire est un greffon osseux comprenant des particules osseuses contenant des cellules osseuses viables ou des cellules ostéogéniques, et le rapport volumique entre la solution de cryoconservation et le greffon osseux est inférieur à 2 ;
iv) la canule comprend en outre une solution de cryoconservation dans son alésage, dans laquelle le greffon tissulaire est un greffon osseux, le greffon osseux comprend des particules osseuses contenant des cellules osseuses viables ou des cellules ostéogéniques, et le greffon osseux ne contient pas de matrice osseuse déminéralisée.

2. La canule selon la revendication 1, dans laquelle la canule présente une densité de particules comprise entre 1,1 et 1,3 g/cm³.

3. La canule selon la revendication 1, dans laquelle la greffe tissulaire est une greffe osseuse cryoconservée, la greffe osseuse comprend de l'os cortical broyé déminéralisé et des fragments osseux non déminéralisés, les fragments osseux comprennent de l'os cortical, cortico-spongieux, spongieux ou des combinaisons de ceux-ci, et les fragments osseux comprennent des cellules ostéogéniques viables.

4. Système destiné à administrer une greffe tissulaire à un site de traitement, comprenant :
(a) la canule selon l'une quelconque des revendications 1 à 3 ; et
(b) un dispositif d'administration comprenant une tige de poussée et une poignée de poussée, dans lequel la tige de poussée est en contact avec la greffe tissulaire et se trouve du côté opposé à l'extrémité distale de celle-ci, et dans lequel la poignée de poussée actionne la tige de poussée pour expulser la greffe tissulaire hors de la canule au niveau de l'extrémité distale vers le site de traitement.

5. Procédé de préparation de la canule selon l'une quelconque des revendications 1 à 3, destinée à acheminer une greffe tissulaire vers un site de traitement, comprenant
(a) le raccordement de la canule à un dispositif d'acheminement, dans lequel le dispositif d'acheminement comprend une tige de poussée ;
(b) la mise en contact de la greffe tissulaire avec la tige de poussée, la tige de poussée se trouvant du côté de la greffe tissulaire opposé à l'extrémité distale.

6. Procédé selon la revendication 5, dans lequel la tige de poussée est configurée pour pousser le greffon tissulaire à une pression ne dépassant pas 275,79 bars (4 000 psi) à la suite de l'étape (b).

7. Procédé de préparation de la canule selon l'une quelconque des revendications 1 à 3 pour l'administration d'un greffon tissulaire à un site de traitement, comprenant
(a) le placement d'une butée tissulaire dans l'alésage à partir de l'extrémité proximale de la canule, de sorte que la butée tissulaire occupe une partie de l'alésage et qu'un espace vide soit formé par la partie restante de l'alésage ;
(b) le chargement d'un premier bouchon tissulaire dans l'espace vide à partir de l'extrémité distale de la canule, le premier bouchon tissulaire ayant un diamètre de section transversale inférieur au diamètre intérieur de la canule ; et
(c) pousser le premier bouchon de tissu vers l'extrémité proximale de l'espace vide, de sorte que le premier greffon tissulaire soit en contact avec la butée tissulaire.

8. Procédé selon la revendication 7, comprenant en outre :
(d) le chargement d'un deuxième bouchon de tissu dans l'espace vide à partir de l'extrémité distale, le deuxième bouchon de tissu ayant un diamètre de section transversale inférieur au diamètre intérieur de la canule ; et
(e) pousser le deuxième bouchon de tissu vers l'extrémité proximale de l'espace vide, de sorte que le deuxième greffon tissulaire soit en contact avec le premier bouchon de tissu, le premier bouchon de tissu et le deuxième bouchon de tissu formant ainsi le greffon tissulaire dans la canule.

9. Procédé selon la revendication 8, comprenant en outre :
(f) charger un troisième bouchon tissulaire dans l'espace vide depuis l'extrémité distale, le troisième bouchon tissulaire ayant un diamètre de section transversale inférieur au diamètre intérieur de la canule ; et
(g) pousser le troisième bouchon tissulaire vers l'extrémité proximale de l'espace vide, de sorte que le troisième bouchon tissulaire soit en contact avec le deuxième bouchon tissulaire, et que le premier bouchon tissulaire, le deuxième bouchon tissulaire et le troisième bouchon tissulaire forment le greffon tissulaire dans la canule.

10. Procédé selon la revendication 9, comprenant en outre la préparation du premier bouchon de tissu et du deuxième bouchon de tissu dans une chambre cylindrique d'un dispositif de chargement, la chambre présentant une finition de surface ne dépassant pas 0,81 micromètre (32 micro-pouces) de valeur efficace.
